# EUROPEAN PATENT APPLICATION

(11) **EP 2 783 721 A1**
(43) Date of publication of application: **01.10.2014**
(21) Application number: 13161203.8
(22) Date of filing: 26.03.2013
(51) Int. Cl.: A61M 5/32

(54) **Needle assembly retainer**

(71) Applicant: Sanofi-Aventis Deutschland GmbH, 65929 Frankfurt am Main (DE)
(72) Inventor: The designation of the inventor has not yet been filed

(57) **Abstract**

Described is a needle assembly retainer (1) comprising a body (2) adapted to hold a needle assembly (10). The needle assembly (10) comprises a hub (11) having an external surface (14) with at least one spline (15) and a needle having a proximal tip (10.1) and a distal tip (10.2). The body (2) has a proximal portion adapted to hold the hub (11) and a distal portion adapted to cover the distal tip (10.2). The needle assembly retainer (1) further comprises a resilient arm (6) formed on the proximal portion and adapted to engage the at least one spline (15) when the needle assembly (10) is rotated relative to the body (2).

## Description

### Technical Field

The invention relates to a needle assembly retainer.

### Background of the Invention

When using a conventional medicament delivery device, such as a pen injector, a new needle assembly must be used for each injection or the patient may be subject to serious risks of infection and/or injection site pain. However, mounting and demounting the needle assembly may lead to risk of needle stick injuries if the tip of the needle is exposed. Needle stick injuries carry the risk of infection and the spread of disease.

Further, patients and/or caregivers may fear that a needle assembly is not properly secured to the delivery device, and over-tigheten the needle assembly. Overtightening can lead to improper engagement of the needle assembly with the delivery device, improper dosing and injury.

Thus, there is a need for a needle assembly retainer which aids in the prevention of injury and infection and ensures proper engagement of the needle assembly with the delivery device.

### Summary of the Invention

It is an object of the present invention to provide an improved needle assembly retainer.

In an exemplary embodiment, a needle assembly retainer according to the present invention comprises a body adapted to hold a needle assembly. The needle assembly comprises a hub having an external surface with at least one spline and a needle having a proximal tip and a distal tip. The body has a proximal portion adapted to hold the hub and a distal portion adapted to cover the distal tip. The needle assembly retainer further comprises a resilient arm formed on the proximal portion and adapted to engage the at least one spline when the needle assembly is rotated relative to the body.

In an exemplary embodiment, the body includes an opening for removing the needle assembly from the body and inserting the needle assembly into the body. The opening is covered by a removable film.

In an exemplary embodiment, the body includes a ledge formed between the proximal portion and the distal portion to support the hub.

In an exemplary embodiment, the arm is adapted to deflect radially relative to the body.

In an exemplary embodiment, the arm includes a hook extending radially inward. The hook includes a ramped surface facing in a first angular direction. The hook includes an abutment surface facing in a second angular direction. The ramped surface engages a spline of the at least one spline to deflect the arm when the needle assembly is rotated in a first rotational direction. At least one of an audible feedback and a tactile feedback is generated when the ramped surface disengages the spline of the at least one spline and the arm returns to a non-deflected position, and the hook engages the hub. The audible feedback is a snap or click sound and the tactile feedback is a decrease in rotational resistance. The abutment surface engages a spline of the at least one spline to prevent the hub from rotating relative to the body in a second rotational direction.

In an exemplary embodiment, the at least one spline includes a chamfered distal end.

In an exemplary embodiment, at least a portion of the body or the resilient arm are made from semi-crystalline materials. The semi-crystalline materials include at least one of Polyamide (PA), Polyoxymethylene (POM), Polybutylene terephthalate (PBT), Polyethylene (PE), or Polypropylene (PP) and thermoplastic elastomers (TPE).

The exemplary embodiments of the needle assembly retainer according to the present invention ensure proper engagement of a needle assembly to a medicament delivery device, and provide feedback to signal such engagement.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a schematic perspective view of an exemplary embodiment of a needle assembly retainer according to the present invention, and
- Figure 2: is a schematic perspective view of an exemplary embodiment of an a needle assembly and a medicament delivery device according to the present invention.

Corresponding parts are marked with the same reference symbols in all figures.

### Detailed Description

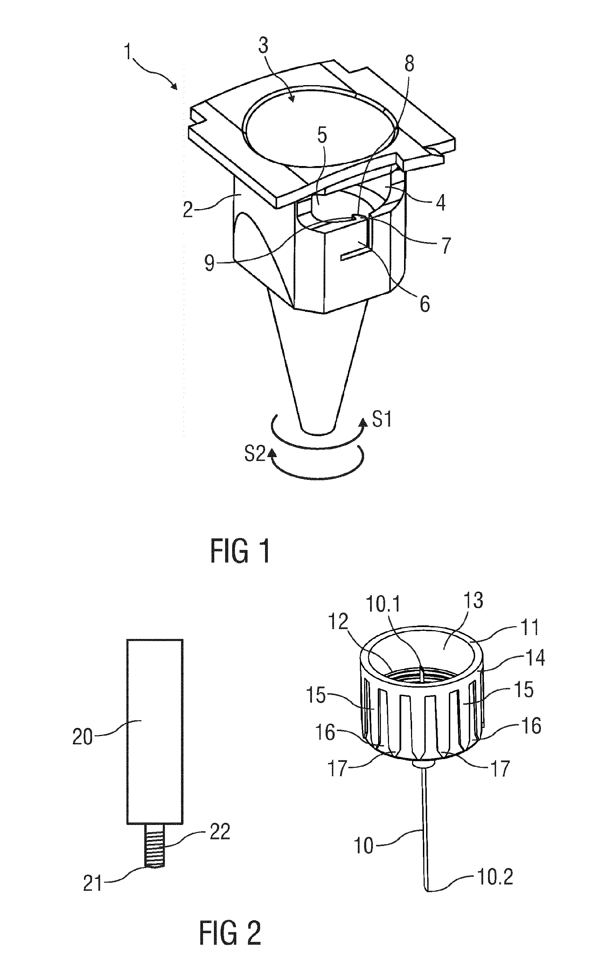

Figure 1 is a schematic perspective view of an exemplary embodiment of a needle assembly retainer 1 according to the present invention. Figure 2 is a schematic perspective view of an exemplary embodiment of a needle assembly 10 and a medicament delivery device 20 according to the present invention.

In an exemplary embodiment, as shown in Figure 2, the needle assembly 10 comprises a hub 11 holding a double tipped needle having a proximal tip 10.1 and a distal tip 10.2. The proximal tip 10.1 is adapted to pierce a septum 21 on the medicament delivery device 20 when the needle assembly 10 is coupled thereto. The distal tip 10.2 is adapted to pierce an injection site. The hub 11 includes a thread 12 formed on an internal surface 13. The thread 12 is adapted to mate with a corresponding thread 22 on the medicament delivery device 20. An external surface 14 of the hub 11 includes a plurality of splines 15 disposed parallel to a longitudinal axis of the hub 11. Adjacent each of the splines 15 is a gap 16, such that the splines and the gaps 16 alternate. In an exemplary embodiment, the splines 15 and gaps 16 are uniformly spaced on the external surface 14 of the hub 11, but those of skill in the art will understand that various sizes, shapes, alignments, and spacings of the splines 15 and gaps 16 may be utilized.

Referring back to the exemplary embodiment of the needle assembly retainer 1 shown in Figure 1, the needle assembly retainer 1 includes a body 2 having a cavity 5 adapted to hold the needle assembly 10. For example, the body 2 includes an opening 3 from removing and inserting the needle assembly 10 from/into the needle assembly retainer 1. A proximal portion of the body 2 is sized and shaped to retainer the hub 11, and a distal portion of the body 2 may be sized and shaped to cover the distal tip 10.2 of the needle. A ledge may be formed between the proximal portion and the distal portion to support the hub 11 within the body 2.

In an exemplary embodiment, the body 2 includes a resilient arm 6 adapted to engage the splines 15 and gaps 16. The arm 6 may be formed on the proximal portion of the body 2 and be capable of deflecting radially relative to the body 2. The arm 6 may include a hook 7 which extends radially into the cavity 5. The hook 7 may have a ramped surface 8 facing in a first angular direction and an abutment surface 9 facing in a second angular direction opposite the first angular direction.

In an exemplary embodiment, the body 2 or any parts thereof may comprise materials with flexible behavior (high strain) combined with sufficient strength in order to facilitate deflection of the arm 6. In an exemplary embodiment such materials may include, but are not limited to, semi-crystalline materials, such as Polyamide (PA), Polyoxymethylene (POM), Polybutylene terephthalate (PBT), Polyethylene (PE), or Polypropylene (PP) or thermoplastic elastomers (TPE).

Prior to use, an unused needle assembly 10 may be stored in the body 2 of the needle assembly retainer 1. The opening 3 may be covered by a peelable film (not shown) to maintain sterility of the needle assembly 10.

After the film has been removed, the delivery device 20 may be inserted through the opening 3 to engage the needle assembly 10. The threads 22 on the delivery device 20 may engage the threads 12 on the hub 11. As the delivery device 20 is rotated relative to the hub 11, the hub 11 is prevented from rotating relative to the body 2, because a spline 15 abuts the ramped surface 8. When the delivery device 20 has properly engaged the hub 11, further rotation of the delivery device 20 will cause the hub 11 to rotate relative to the body 2. At this point, sufficient rotational force is applied to the hub 11 by the delivery device 20 such that the spline 15 engages the ramped surface 8, causing the arm 6 to deflect radially. After the spline 15 is bypassed, the arm 6 returns to a non-deflected position. An audible feedback (e.g., click or snap sound) may be provided when the arm 6 returns the non-deflected position, because the hook 7 may impact the hub 11. The audible feedback signals that the needle assembly 10 is properly engaged to the delivery device 20 and no further rotation/torque is necessary. Additionally, a tactile feedback may be provided, because an increased resistance to rotation of the delivery device 20 may be felt as the spline 15 deflects the arm 6 and when the spline 15 bypasses the arm the resistance is released.

After the needle assembly 10 has been used for an injection, it may be returned to the needle assembly retainer 1. When the used needle assembly 10 is inserted into the body 2, the hook 7 may engage a spline 15 or a gap 16. If the hook 7 engages a spline 15, the arm 6 may be deflected radially. If the hook 7 engages a gap 16, the arm 6 may remain in the non-deflected position. In another exemplary embodiment, the splines 15 may include chamfered distal ends to direct the hook 7 into an adjacent gap 16 when the needle assembly 10 is inserted into the body 2.

When the delivery device 20 and the needle assembly 10 attached thereto are rotated relative to the body 2, a spline 15 on the hub 11 will abut the abutment surface 9 on the hook 7, preventing further rotation of the needle assembly 10 relative to the body 2. Because the needle assembly 10 is prevented from rotating relative to the body 2, continued rotation of the delivery device 20 will cause the delivery device 20 to disengage the needle assembly 10.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle assembly retainer (1) comprising:
a body (2) adapted to hold a needle assembly (10), the needle assembly (10) comprising a hub (11) having an external surface (14) with at least one spline (15) and a needle having a proximal tip (10.1) and a distal tip (10.2), the body (2) having a proximal portion adapted to hold the hub (11) and a distal portion adapted to cover the distal tip (10.2);
a resilient arm (6) formed on the proximal portion and adapted to engage the at least one spline (15) when the needle assembly (10) is rotated relative to the body (2).

2. The needle assembly retainer (1) according to claim 1, wherein the body (2) includes an opening (3) for removing the needle assembly (10) from the body (2) and inserting the needle assembly (10) into the body (2).

3. The needle assembly retainer (1) according to claim 2, wherein the opening (3) is covered by a removable film.

4. The needle assembly retainer (1) according to any one of the preceding claims, wherein the body (2) includes a ledge formed between the proximal portion and the distal portion to support the hub (11).

5. The needle assembly retainer (1) according to any one of the preceding claims, wherein the arm (6) is adapted to deflect radially relative to the body (2).

6. The needle assembly retainer (1) according to any one of the preceding claims, wherein the arm (6) includes a hook (7) extending radially inward.

7. The needle assembly retainer (1) according to claim 6, wherein the hook (7) includes a ramped surface (8) facing in a first angular direction.

8. The needle assembly retainer (1) according to claim 7, wherein the hook (7) includes an abutment surface (9) facing in a second angular direction.

9. The needle assembly retainer (1) according to claim 8, wherein the ramped surface (8) engages a spline (15) of the at least one spline (15) to deflect the arm (6) when the needle assembly (10) is rotated in a first rotational direction.

10. The needle assembly retainer (1) according to claim 9, wherein at least one of an audible feedback and a tactile feedback is generated when the ramped surface (8) disengages the spline (15) of the at least one spline (15) and the arm (6) returns to a non-deflected position, and the hook (7) engages the hub (11).

11. The needle assembly retainer (1) according to claim 9, wherein the audible feedback is a snap or click sound and the tactile feedback is a decrease in rotational resistance.

12. The needle assembly retainer (1) according to claim 9, wherein the abutment surface (9) engages a spline (15) of the at least one spline (15) to prevent the hub (11) from rotating relative to the body (2) in a second rotational direction.

13. The needle assembly retainer (1) according to any one of the preceding claims, wherein the at least one spline (15) includes a chamfered distal end.

14. The needle assembly retainer (1) according to any one of the preceding claims, wherein at least a portion of the body (2) or the resilient arm (6) are made from semi-crystalline materials.

15. The needle assembly retainer (1) according to claim 13, wherein the semi-crystalline materials include at least one of Polyamide (PA), Polyoxymethylene (POM), Polybutylene terephthalate (PBT), Polyethylene (PE), or Polypropylene (PP) and thermoplastic elastomers (TPE).
